# EUROPEAN PATENT SPECIFICATION

(11) **EP 1 283 062 B1**
(45) Date of publication and mention of the grant of the patent: **03.05.2006**
(21) Application number: 01830528.4
(22) Date of filing: 07.08.2001
(51) Int. Cl.: A61L 9/03, A01M 1/20

(54) **Polyfunctional electric wall evaporator**
Elektrischer polyfunktioneller Wandsteckdosenverdampfer
Evaporateur électrique murale polyfonctionnel

(43) Date of publication of application: 12.02.2003
(73) Proprietor: S.C. JOHNSON & SON, INC., Racine, WI 53403-2236 (US)
(72) Inventor: Pedrotti, Andrea, 38070 Pietromurata (IT); Campedelli, Paolo, 38065 Mori (IT); Zobele, Franco, 38100 Trento (IT); Baldessari, Stefano, 38052 Caldonazzo (IT)
(74) Representative: Ruschke, Hans Edvard

(56) References cited:
- EP-A- 0 962 132
- WO-A-98/46284
- US-A- 5 647 053
- US-A- 5 932 147

## Description

The present invention relates to a multiple-function electric wall vaporizer for aromatic or insecticide liquid substances, i.e. to a vaporizer which is intended to be used directly plugged into an electric wall socket and which, in addition to the basic function of vaporization of the liquid substance by means of an electric heating device, may be equipped with multiple functions, both additional and alternative, so as to allow easier or more sophisticated use of the vaporizer.

The world market of vaporizers for aromatic (such as perfumes, deodorants and the like) or insecticide substances is, as is well-known to specialists in the sector, extremely varied both from the point of view of the technical specifications of the electric parts of the vaporizer and from the point of view of the requirements of the users. For example, U.S. Patent No. 5647053 discloses a vapor dispensing device for insecticides or perfumes having an outer shell having vapor dispensing opening, a one piece electric plug heater block having attached electric plug pins, a container such as a bottle sealed with closure which holds a wick. The electric plug heater block described in said patent is rotatably attached to the outer shell such that the block can rotate through predefined range of rotation around an axis parallel to the plug pins.

The European Patent EP0962132 discloses an electric evaporator for insecticides or perfumes comprising a housing, a revolving plug, an electric heating device and a bottle containing the liquid formulation provided with a wick. The patent relates to an electric evaporator for liquid formulations contained in bottles having a non-circular section wherein it may be possible to continuously change the position of the wick for the liquid formulation, in respect of the heating device of the evaporator.

The U.S. Patent No. 5932147 discloses an air freshener device comprising a housing having air input and output ports, an air driven rotatable fan disposed in said housing and an air freshener pellet disposed within said housing.

WO9846284 discloses an electric wall air freshener dispenser device comprising a disposable cartridge with a reservoir chamber of liquid air freshener medium, an elongated wick and a plug-in heater module. This variation in the market gives rise to considerable problems for the manufacturers of these apparatuses, who in fact, on the one hand, must produce different models for each specific market requirement and, on the other hand, must continuously reduce production costs in order to keep pace with international competition.

As regards the technical specifications of the electric components, the problem does not lie so much in the safety standards of said components, since it is sufficient to adopt, as a standard, the most stringent regulations from among those existing in the various countries; the savings which result from a more homogeneous production of the electric components in fact abundantly offset the increased cost of higher quality components even where these are not strictly necessary. A more important problem is rather that of the plugs for said apparatuses, for which it has not been possible to achieve hitherto - nor certainly will it be possible in the short term - an international or even European standard, since such a standardisation would mean a reorganisation of the entire electrical domestic and office network on a national level.

However a problem even greater than that of the power supply plugs is that relating to the various possible additional functions with which a vaporizer may be provided, depending on the preferences and the economic resources of the individual markets. In fact, whereas in the most recently developed countries, are strongly requested vaporizers having only the basic functions - i.e. that of power supply and heating and, in case, a few simple additional functions, such an operating lamp, a switch or an electric socket for replacing the one occupied by the vaporizer - in the more advanced or richer countries, the clients' requirements are greater and more complex such that there is a demand, for example, for vaporizers which are equipped with a fan, are operating programmer, a night light or other more sophisticated functions of this type.

It must furthermore be noted that also this type of apparatus, like many other mass-consumption products, is now influenced by fashion; there is therefore the need to provide apparatuses provided with an aesthetic appearance which is not only attractive from the point of view of design or colour, but which can also be modified easily and at a low cost, so as to remain appealing vis-à-vis the preferences of the constantly evolving market.

Hitherto, as mentioned above, the response of vaporizer manufacturers has been to produce and keep on catalogue a very large number of apparatuses which vary from each other, not only with regard to the intrinsic technical characteristics of the apparatus, but in particular as regards the various additional functions with which they are equipped and their different design.

This production policy, however, does not allow a reduction in the production costs of vaporizers below a well-defined limit, this being incompatible, however, with the desire to achieve a further expansion of the market.

After studying this problem in depth, the inventors of the present invention have realised that this problem could possibly be solved in a satisfactory manner by merely making a radical change to the design approach adopted hitherto and by therefore developing a new type of vaporizer which, irrespective of the functional and aesthetic requirements of the individual apparatus, is able to accommodate, within a single technical structure, several functions and several possible external designs, without the use of any one of said functions or said designs requiring any modification to said technical structure.

On the basis of this initial intuition, the Applicant has therefore developed the multiple-function vaporizer for aromatic or insecticide liquid substance according to the present invention, having the general characteristic features indicated in Claim 1 and in the dependent Claims 2 to 13.

The present invention will anyhow be illustrated in more detail in the following, with reference to the abovementioned various embodiments illustrated solely by way of example in the views of the accompanying drawings, in which:
Figs. 1 and 2 are respectively rear and front perspective views of a first embodiment of the vaporizer according to the present invention;
Fig. 3 is an exploded view of the vaporizer according to Figs. 1 and 2, with a sliding-contact plug, provided only with basic functions;
Fig. 4 is a view, similar to that of Fig. 3, in which the vaporizer is also provided with the electric socket additional function;
Fig. 5 is a view, similar to that of Fig. 4, in which the vaporizer is also provided with the fan additional function;
Fig. 6 is a view, similar to that of Fig. 4, from a different viewpoint, in which the vaporizer is also provided with the night light additional function;
Fig. 7 is a view similar to that of Fig. 6, in which the vaporizer is provided with the operating programmer additional function as an alternative to the night light function;
Fig. 8 illustrates a second embodiment of the vaporizer according to the present invention, with a flexible-cable plug, provided with the basic functions and with the draught regulator additional function, where, for the sake of simplicity, illustration of the third outer front shell has been omitted;
Fig. 9 illustrates the vaporizer according to Fig. 8, also provided with the electric socket additional function;
Fig. 10 illustrates the vaporizer according to Fig. 9, also provided with the operating lamp additional function;
Fig. 11 illustrates a third embodiment of the vaporizer according to the present invention, with a flexible-cable plug, having the basic functions and the additional functions of draught regulator, electric socket and fan, where, for the sake of simplicity, illustration of the third outer front shell has been omitted;
Fig. 12 illustrates a vaporizer similar to that illustrated in Fig. 11, in which the fan additional function is replaced by the night light additional function; and
Fig. 13 shows a vaporizer similar to that illustrated in Fig. 11, in which the fan additional function is replaced by the operating programmer additional function.

The various embodiments which are illustrated in the above drawings and which will be described in the following in a more detailed way, obviously all have only the purpose of illustrating the inventive idea of the present invention. This inventive idea, in fact, is not linked to the individual additional functions present in the vaporizer or to their physical arrangement inside the two shells, but rather to the fact of providing a vaporizer in which a hidden, multiple-function, internal, technical structure consisting of two combined shells provided with a variable number of additional functions is associated with an on sight external shell provided with access windows only for said variable number of additional functions.

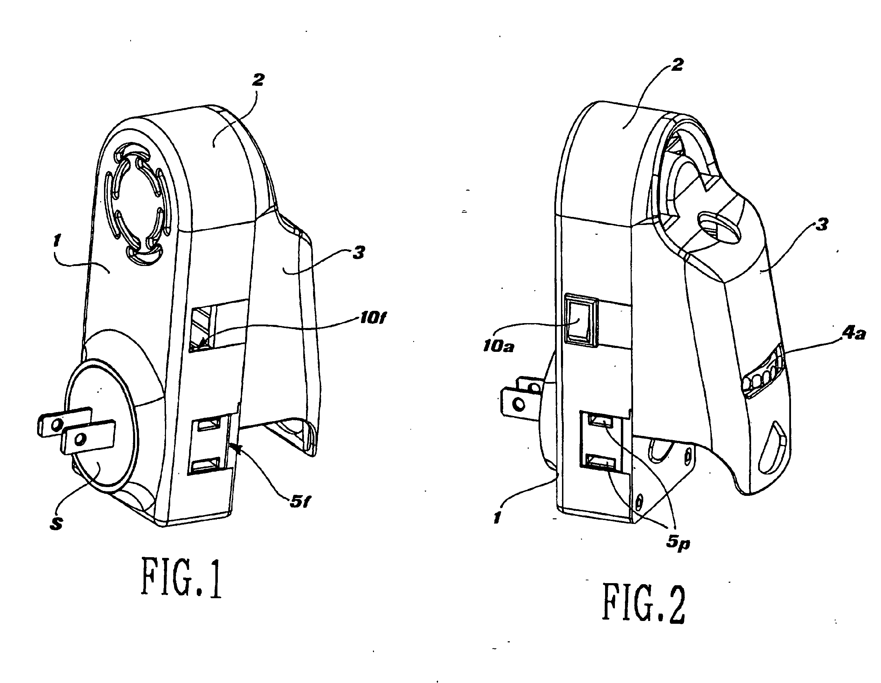

Figs. 1 to 7 illustrate a first embodiment of the vaporizer according to the present invention which shows a first shell 1 and a second shell 2 which can be joined together in a manner known per se and which together form the technical structure of the vaporizer. These shells comprise both the basic functions of the vaporizer and one or more additional functions. In particular the basic functions comprise: an electric plug S of the rotating type and a contact carrier M comprising several contacts, which are both stably fixed to the shell 1; an electric heating device R connected to the contacts M_{R} of the terminal block M and a bottle F containing the substance to be evaporated with the relative wick W, the housings for these elements being instead formed in the shell 2. The plug S is of the sliding-contact type with the contacts S_{M} of the plug S which engage with two possible corresponding pairs of contacts M_{S} of the contact carrier M which are mutually offset by 90°, allowing a rotation of the plug through 360° and therefore easy adaptation of the plug itself to all the socket systems in use (UK or Austratian fixed plugs or Argentinian plugs with rotation through 270°).

According to a fundamental feature of the present invention, the vaporizer is finally completed by a third shell 3 which completely surrounds the outer surface of the shell 2 and which therefore forms the whole, substantially on sight, part of the vaporizer since the outer part of the shell 1 is located next to the wall of the electric socket used, while the on sight side part of the shell 2 does not have any important aesthetic function. Said third shell 3 may be designed with the maximum freedom from an aesthetic point of view, since the sole functional requirements which it must satisfy is that of being provided with means for fastening to the shell 2 in predetermined positions and with access windows for the sole functions which are used with that particular third shell.

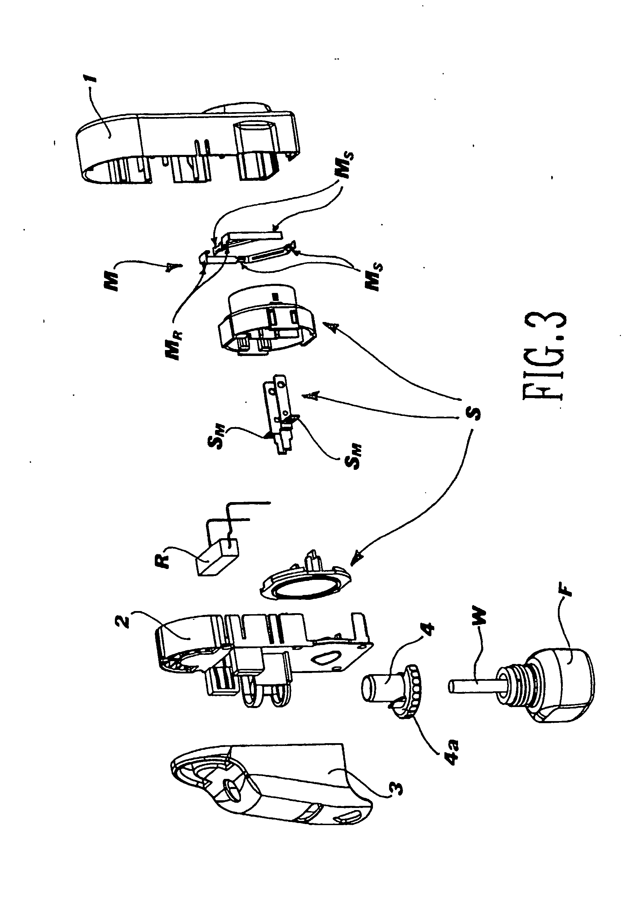

In the first embodiment of the vaporizer according to the present invention, several additional functions may be associated to the basic functions described above and illustrated in Fig. 3, without any modification to the structure of the shells 1 and 2 nor to the configuration of the basic functions; these latter in fact are already initially designed in view of the multiple usage of the vaporizer.

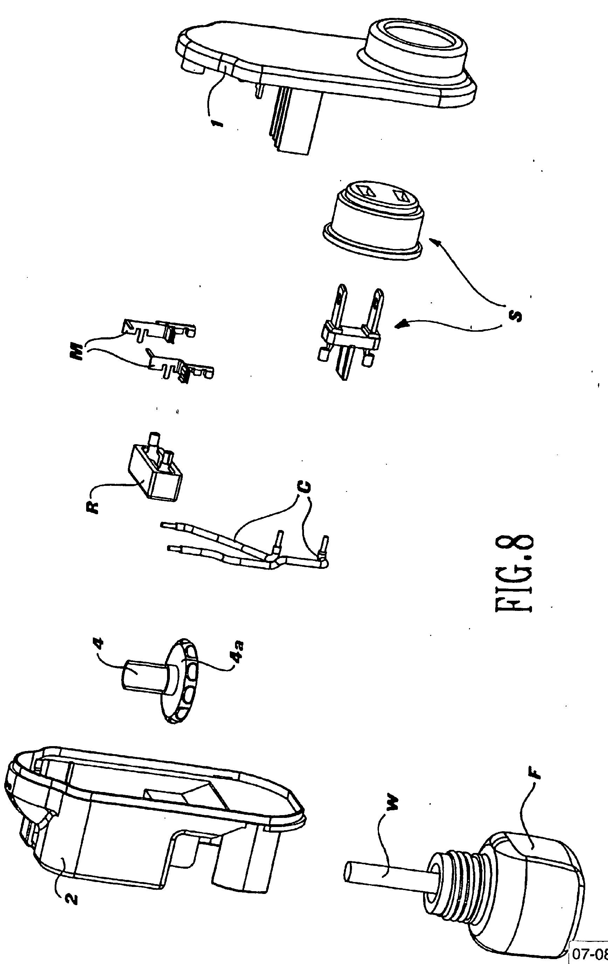

Fig. 3 illustrates the draught regulator additional function. This function is obtained by means of a tube 4 which has a partially removed wall (as more clearly visible in Fig. 8) provided with an operating wheel 4a. The tube 4 and the wheel 4a are housed inside the shell 2 in a position such that the tube 4 can house the wick W internally and the wheel 4a can be actuated on the outside of the shell 3. By rotating the wheel 4a, the chimney effect produced by heating of the device R is varied and therefore the quantity of active substance introduced into the environment per unit of time is varied. The draught regulator function may also be obtained by using cam devices - including those of a type known per se (not shown in the drawings) - which produce the lateral displacement of the wick, thus allowing its distance from the heating device to be adjusted. The two types of devices indicated above may be used as an alternative to each other or also jointly when it is required to obtain the possibility of a more sophisticated regulation.

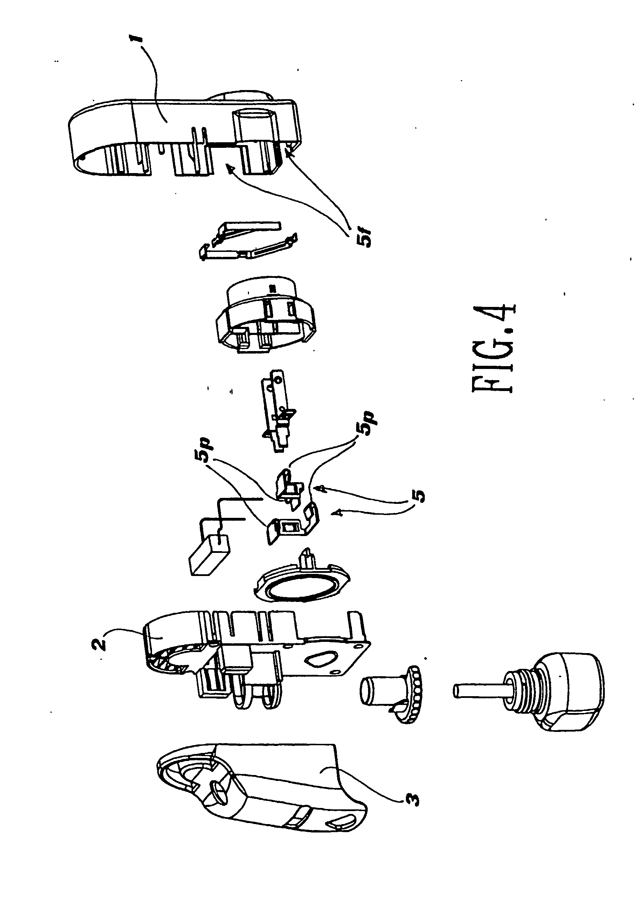

Fig. 4 illustrates the electric socket additional function. This function is obtained by means of a pair of metal strips 5 which are housed inside the body of the rotating plug S. One end of the strips 5 is apt to be press-fitted into the spring contacts S₅ of the pins of the plug S, while the other end, which has a clamping system 5p for an electric plug, is accessible via either of two windows 5f provided in the shell 1, depending on the position assumed by the plug S.

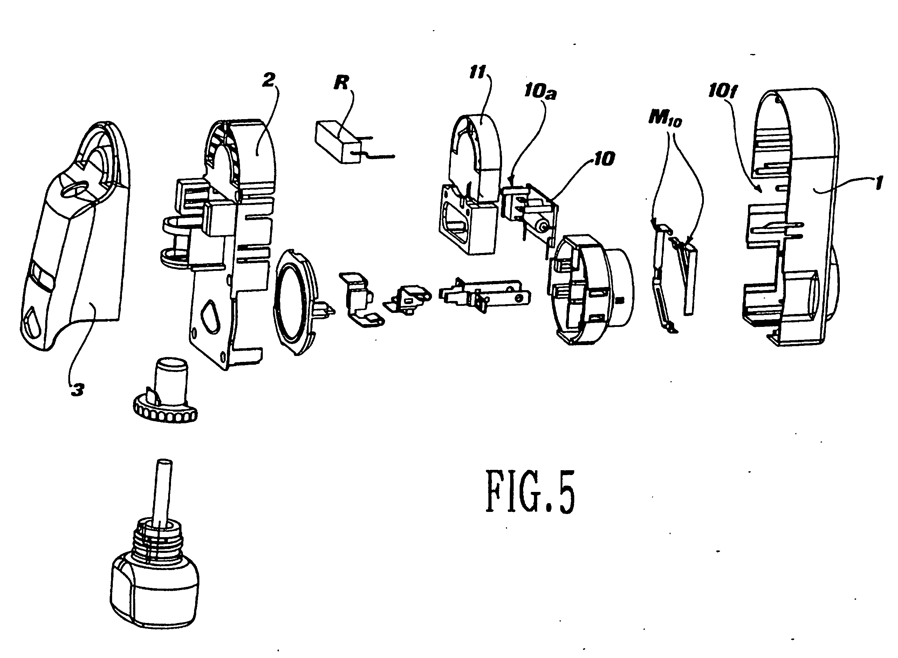

Fig. 5 illustrates the fan additional function. This consists of a more complex function which is housed in the top part of the shells 1 and 2 which are provided, for this purpose, with a wide cavity. This cavity houses a printed electric circuit 10 and a fan assembly 11 which is powered and controlled thereby. The circuit 10 is in turn powered by means of the contacts M₁₀ (which are precisely the same contacts M_{R} described further above, while the heating device R is in this case connected to the circuit 10 instead of directly to the contact carrier M. The circuit 10 is equipped with a switch 10a which is housed inside the window 10f of the shell 1.

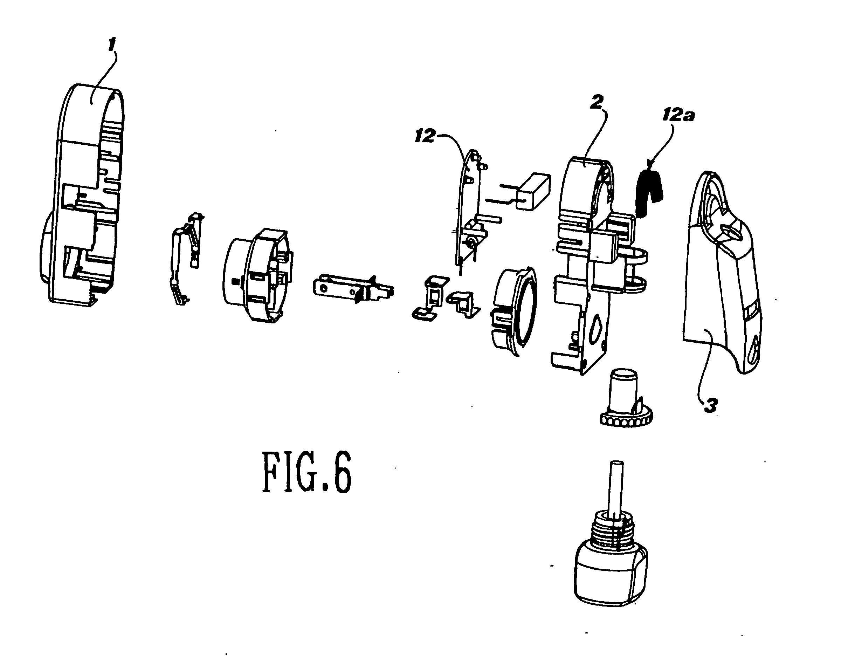

Fig. 6 illustrates the night light 12 additional function, which light may be chosen from among the various known types currently present on the market, such as incandescent lamps, neon lamps or LED devices (such as those illustrated in the drawing). The light emitted by said light devices may be usefully diffused by means of a diffusing lens 12a. The electric connections are identical to those illustrated with regard to Fig. 5.

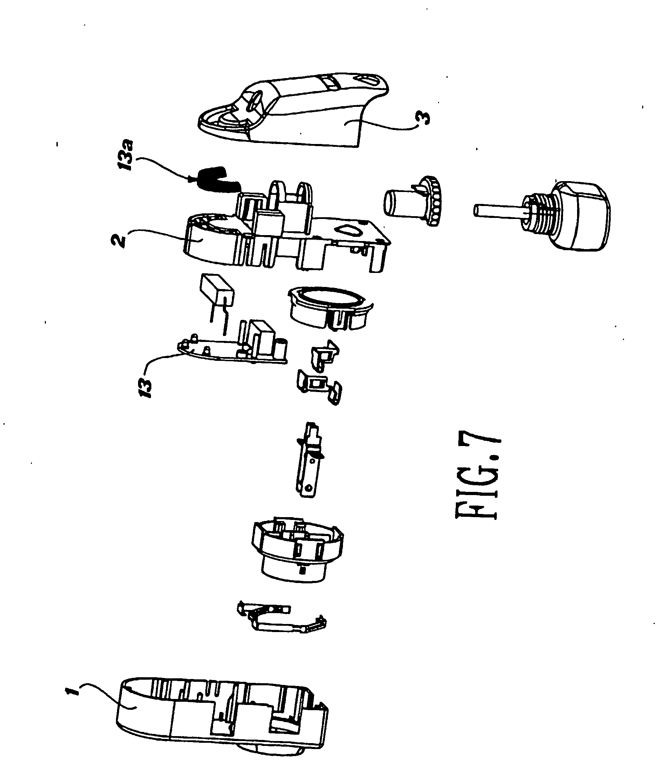

Fig. 7 illustrates the operating programmer 13 additional function. In this case the LED devices have a much lower power since they do not have the function of illuminating, but only that of signalling the different operating modes of the vaporizer. A diffuser 13a may in any case be used to make these signals more visible. In this case also, the electrical connections are identical to those illustrated with regard to Fig. 5.

From the above description it should be clear that it is possible to modify the number and the type of additional functions of the vaporizer without making any modification to its technical structure, consisting of the shells 1 and 2, but by simply preparing for each given vaporizer design a respective third shell 3 having the desired aesthetic characteristics and only those access windows which are necessary for accessing to the particular functions to be used in that design. During assembly, said functions are easily inserted into the respective seats and electric contacts already provided in the abovementioned shells 1 and 2. The object of the invention is therefore fully achieved.

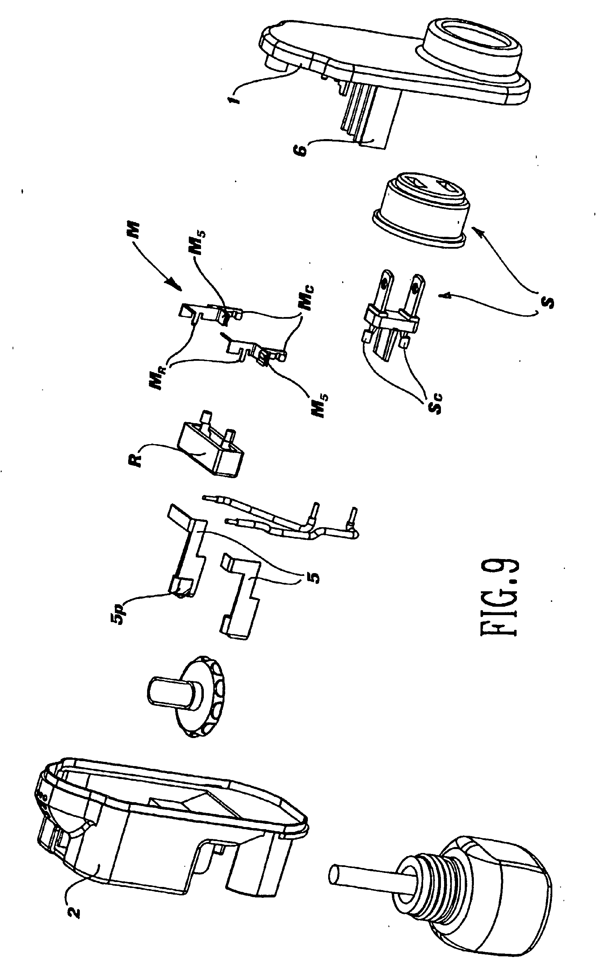

Figs. 8 to 10 illustrate a second embodiment of the vaporizer according to the present invention which comprises elements similar to those described with respect to the functions of the first embodiment and which will therefore be identified by the same reference numbers and letters. This embodiment therefore clearly shows the first shell 1 and the second shell 2 which joined form the technical structure of the vaporizer. The shell 3, on the other hand, is not shown so that the drawing may be more easily understood. The same basic functions seen above are associated with the shells 1 and 2, namely: electric plug S of the rotating type and contact carrier M fixed to the shell 1; electric heating device R and bottle F for the substance to be evaporated with relative wick W, the housings for which are formed in the shell 2. The plug S is in this embodiment of the type comprising flexible electric cables C which connect the contacts Sc of the plug S (Fig. 9) to the contacts Mc of the contact carrier M. In the second embodiment here illustrated, up to three additional functions may be associated with these basic functions.

Fig. 8 illustrates the draught regulator additional function already illustrated above.

Fig. 9 illustrates the electric socket additional function. This function, also in this embodiment, is obtained by means of the pair of metal strips 5 which are now housed on the supports 6 of the shell 1. One end of the strips 5 is apt to be press-fitted into the spring contacts M₅ of the contact carrier M, while the other end, which has a clamping system 5p for an electric plug, is accessible via a special window provided in the front of the shell 2.

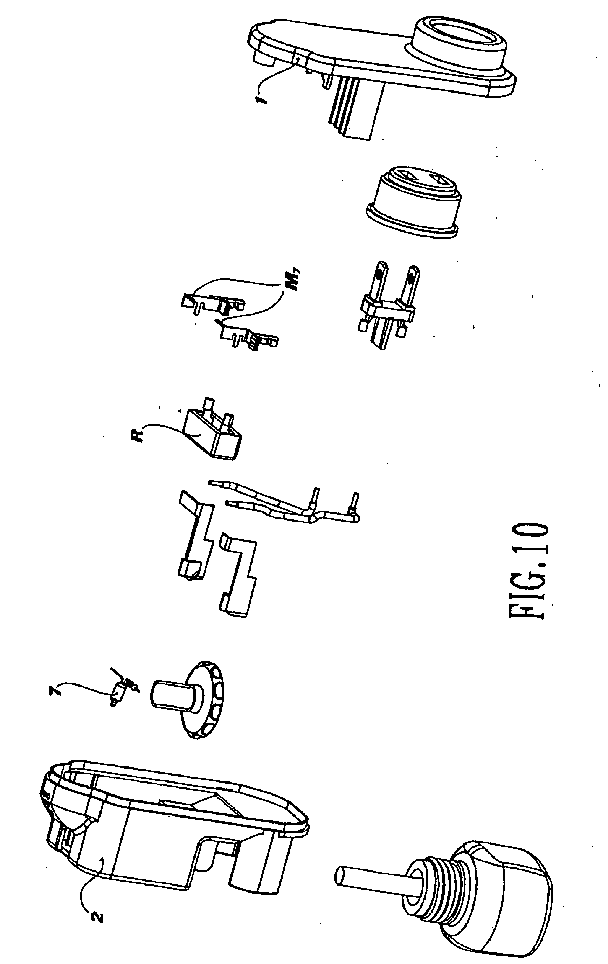

Fig. 10 illustrates the operating lamp additional function. This function is obtained by means of a neon lamp 7 housed in the shell 2 at a respective window. The contacts of the lamp 7 are apt to be press-fitted into the spring contacts M₇ of the contact carrier M.

For the sake of convenience and clarity of illustration, the various additional functions described above have been shown in a sequentially incremental manner from Fig. 8 to Fig. 10, but it is clear that the vaporizer according to the invention may be equipped with any number of these additional functions which may be inserted or removed from the vaporizer entirely as desired, none of said functions being electrically or mechanically dependent on the others.

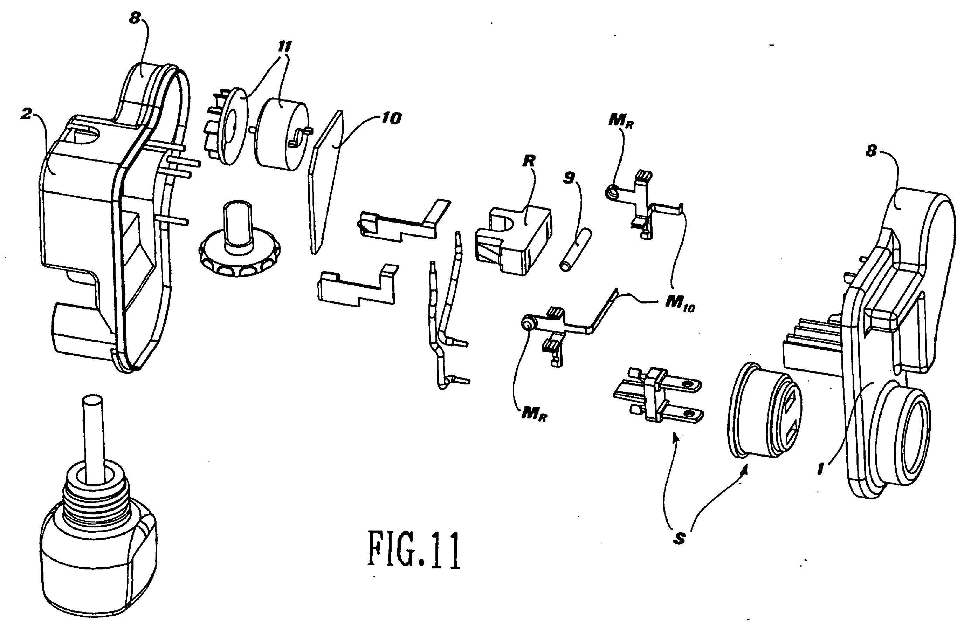

Finally, Figs. 11 to 13 illustrate a third embodiment of the invention which also comprises elements which are similar to those described with respect to the functions of the first and second embodiment and which will therefore be identified by the same reference numbers and letters. The shells 1 and 2 in this case have a lateral appendage 8 which may house the more complex and therefore more bulky additional functions which in the first embodiment were housed in the top part of the shells 1 and 2.

The heating device R has a different configuration, having a projecting U-shaped part apt to surround the wick W, while the relative contacts M_{R} of the contact carrier M are of the type apt to be inserted into a special cavity of the device R and to retain between them the resistor 9 in a stable and resilient manner. In any case these consist of devices which are well-known to the person skilled in the art and which therefore will not be further described in greater detail.

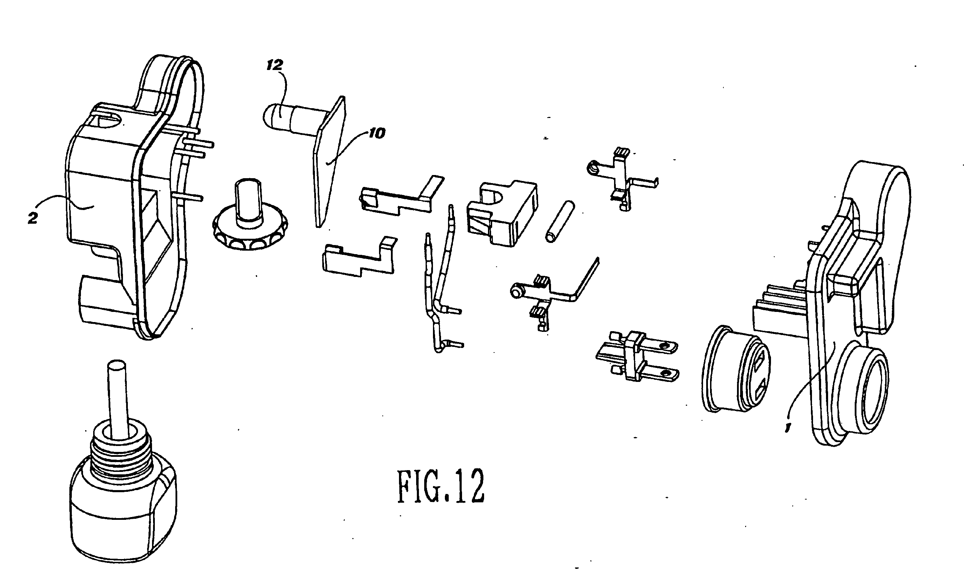

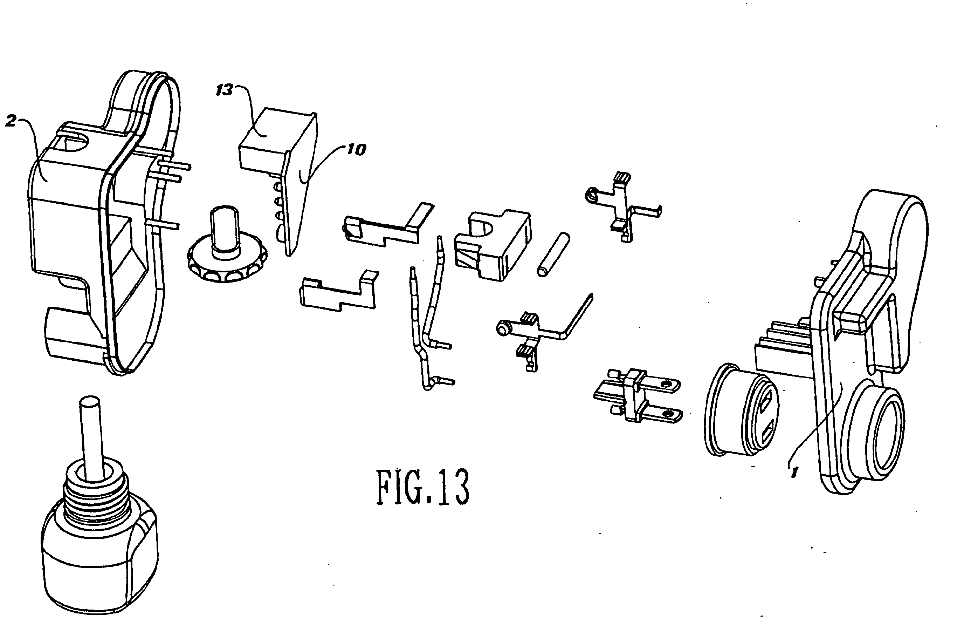

In this third embodiment, the vaporizer comprises, in addition to the basic functions, the draught regulator and electric socket additional functions already described previously. As stated above, it is then envisaged that a further, more complex additional function may be housed inside the appendage 8. This additional function may be that of the fan 11 (Fig. 11), the night light 12 (Fig. 12), shown here as a neon lamp, and finally the operating programmer 13 (Fig. 13). In all cases this additional function is powered by means of a printed electric circuit 10 which is housed in the bottom of the appendage 8 of the shell 1 and is powered by the contacts M₁₀.

From the above description it should be obvious how the vaporizer according to the present invention has fully achieved the desired object. With the said vaporizer, in fact, it is possible to provide a single multiple-function technical structure consisting of the assembly of shells 1 and 2 and to equip this technical structure with the basic functions and the desired number and type of additional functions without this basic structure having to be altered or modified. Finally this technical structure may be "dressed" with a single outer shell 3 intended to constitute substantially the sole on sight part of the vaporizer and having, formed in itself, only the windows for accessing to the additional functions provided for the particular concerned vaporizer.

## Claims

1. Multiple-function electric wall vaporizer for aromatic or insecticide liquid substances of the type in which a support container houses at least one bottle provided with a wick for the liquid substance, an electric heating device apt to heat said wick and an electric circuit for powering the heating device ending in a plug for connection to the electric mains, **characterized in that** said container is formed by:
- a first rear shell and a second front shell which are joined together and which house at least the power supply plug, the electric circuit connected thereto, the bottle containing the substance to be vapprized and said heating device and in which are also provided housings and/or access windows for one or more additional functional devices; and
- a third, outer, front shell, having an aesthetic function, combined with said first or second shell and apt to cover them at least partly and provided with access windows corresponding to one or more of the access windows provided in said first or second shell.

2. Multiple-function electric vaporizer as claimed in Claim 1), wherein said first rear shell houses at least the power supply plug and the electric circuit connected thereto and said second, inner, front shell houses at least said bottle and said heating device.

3. Multiple-function electric vaporizer as claimed in Claim 1) or 2), wherein said electric circuit also comprises fast-engagement connections for powering the additional, electrically powered, functional devices.

4. Multiple-function electric vaporizer as claimed in any one of Claims 1) to 3), wherein said electric circuit comprises flexible cables for connection to said plug.

5. Multiple-function electric vaporizer as claimed in any one of Claims 1) to 3), wherein said electric circuit comprises sliding contacts for connection to said plug, apt to allow rotation of the plug through 360°.

6. Multiple-function electric vaporizer as claimed in any one of the preceding claims, wherein said additional functional device is a draught regulator.

7. Multiple-function electric vaporizer as claimed in any one of the preceding claims, wherein said additional functional device is a device able to laterally displace the wick towards the heating device.

8. Multiple-function electric vaporizer as claimed in any one of the preceding claims, wherein said additional functional device is an electric socket.

9. Multiple-function electric vaporizer as claimed in any one of the preceding claims, wherein said additional functional device is an operating lamp.

10. Multiple-function electric vaporizer as claimed in any one of the preceding claims, wherein said additional functional device is a fan.

11. Multiple-function electric vaporizer as claimed in any one of the preceding claims, wherein said additional functional device is a night light.

12. Multiple-function electric vaporizer as claimed in any one of the preceding claims, wherein said additional functional device is an operating programmer.

13. Multiple-function electric vaporizer as claimed in any one of the preceding claims, wherein said additional functional device is a switch for switching on the vaporizer.

## Patentansprüche

1. Multifunktionaler elektrischer Wand-Verdampfer für aromatische oder insektizide Flüssigsubstanzen, dessen Stützbehälter mindestens eine Flasche mit einem Docht für die Flüssigsubstanz, eine elektrische Heizeinrichtung, mit der der Docht beheizbar ist, und eine elektrische Schaltung aufweist, die die Heizeinrichtung speist und zu einem Stecker zum Anschluss an das elektrische Stromnetz ausläuft, **dadurch gekennzeichnet, dass** der Behälter gebildet wird von:
- einer ersten hinteren und einer zweiten vorderen Schale, die zusammengefügt sind und die mindestens den Stromstecker, die mit diesem verbundene elektrische Schaltung, die die zu verdampfende Substanz enthaltende Flasche und die Heizeinrichtung aufnehmen, und in denen auch Aufnahmen und/oder Zugangsfenster für eine oder mehrere Zusatzfunktionseinrichtungen vorgesehen sind; und
- einer dritten äußeren vorderen Schale mit ästhetischer Funktion, die mit der ersten oder der zweiten Schale kombiniert ist, diese mindestens teilweise abdecken kann und mit Zugangsfenstern entsprechend dem einen oder den mehreren Zugangsfenstern in der ersten oder zweiten Schale versehen ist.

2. Multifunktionaler elektrischer Verdampfer nach Anspruch 1, bei dem die erste hintere Schale mindestens den Stromstecker und die an diesen angeschlossene elektrische Schaltung und die zweite innere Schale mindestens die Flasche und die Heizeinrichtung aufnehmen.

3. Multifunktionaler elektrischer Verdampfer nach Anspruch 1 oder 2, bei dem die elektrische Schaltung auch Schnellverbindungen zur Speisung der elektrisch betriebenen funktionalen Zusatzeinrichtungen aufweist.

4. Multifunktionaler elektrischer Verdampfer nach einem der Ansprüche 1 bis 3, bei dem die elektrische Schaltungen flexible Leitungen zur Verbindung mit dem Stecker aufweist.

5. Multifunktionaler elektrischer Verdampfer nach einem der Ansprüche 1 bis 3, bei dem die elektrische Schaltung Gleitkontakte zur Verbindung mit dem Stecker aufweist und der Stecker über 360° verdrehbar ist.

6. Multifunktionaler elektrischer Verdampfer nach einem der voranstehenden Ansprüche, bei dem die funktionale Zusatzeinrichtung ein Zugeinsteller ist.

7. Multifunktionaler elektrischer Verdampfer nach einem der voranstehenden Ansprüche, bei dem die funktionale Zusatzeinrichtung eine Einrichtung ist, mit der der Docht seitlich zur Heizeinrichtung hin versetzbar ist.

8. Multifunktionaler elektrischer Verdampfer nach einem der voranstehenden Ansprüche, bei dem die funktionale Zusatzeinrichtung eine elektrische Steckfassung ist.

9. Multifunktionaler elektrischer Verdampfer nach einem der voranstehenden Ansprüche, bei dem die funktionale Zusatzeinrichtung eine Betriebsmeldelampe ist.

10. Multifunktionaler elektrischer Verdampfer nach einem der voranstehenden Ansprüche, bei dem die funktionale Zusatzeinrichtung ein Gebläse ist.

11. Multifunktionaler elektrischer Verdampfer nach einem der voranstehenden Ansprüche, bei dem die funktionale Zusatzeinrichtung ein Nachtlicht ist.

12. Multifunktionaler elektrischer Verdampfer nach einem der voranstehenden Ansprüche, bei dem die funktionale Zusatzeinrichtung ein Programmschalter ist.

13. Multifunktionaler elektrischer Verdampfer nach einem der voranstehenden Ansprüche, bei dem die funktionale Zusatzeinrichtung ein Schalter zum Einschalten des Verdampfers ist.

## Revendications

1. Vaporisateur mural électrique multifonctions pour des substances liquides aromatiques ou insecticides du type dans lequel un réceptacle de support renferme au moins une bouteille pourvue d'une mèche pour la substance liquide, un dispositif de chauffage électrique capable de chauffer ladite mèche et un circuit électrique destiné à alimenter le dispositif de chauffage qui se termine par une fiche pour raccordement au réseau électrique, **caractérisé en ce que** ledit réceptacle est formé par :
- un premier boîtier arrière et un deuxième boîtier avant qui sont reliés ensemble et qui renferment au moins la fiche d'alimentation, le circuit électrique qui y est raccordé, la bouteille contenant la substance devant être vaporisée et ledit dispositif de chauffage et dans lesquels sont prévus des logements et/ou des fenêtres d'accès pour un ou plusieurs dispositifs fonctionnels additionnels; et
- un troisième boîtier avant extérieur, ayant une fonction esthétique, combiné audit premier ou deuxième boîtier et capable de les recouvrir au moins partiellement et pourvu de fenêtres d'accès correspondant à une ou plusieurs des fenêtres d'accès prévues dans ledit premier ou deuxième boîtier.

2. Vaporisateur mural électrique multifonctions selon la revendication 1, dans lequel ledit premier boîtier arrière renferme au moins la fiche d'alimentation et le circuit électrique relié à celle-ci et ledit deuxième boîtier avant interne renferme au moins ladite bouteille et ledit dispositif de chauffage.

3. Vaporisateur mural électrique multifonctions selon la revendication 1 ou 2, dans lequel ledit circuit électrique comprend également des connexions à engagement rapide afin d'alimenter les dispositifs fonctionnels additionnels à alimentation électrique.

4. Vaporisateur mural électrique multifonctions selon l'une quelconque des revendications 1 à 3, dans lequel ledit circuit électrique comprend des câbles flexibles pour raccordement à ladite fiche.

5. Vaporisateur mural électrique multifonctions selon l'une quelconque des revendications 1 à 3, dans lequel ledit circuit électrique comprend des contacts coulissants pour raccordement à ladite fiche, qui peuvent permettre une rotation de la fiche sur 360°.

6. Vaporisateur mural électrique multifonctions selon l'une quelconque des revendications précédentes, dans lequel ledit dispositif fonctionnel additionnel est un régulateur de soutirage.

7. Vaporisateur mural électrique multifonctions selon l'une quelconque des revendications précédentes, dans lequel ledit dispositif fonctionnel additionnel est un dispositif capable de déplacer latéralement la mèche vers le dispositif de chauffage.

8. Vaporisateur mural électrique multifonctions selon l'une quelconque des revendications précédentes, dans lequel ledit dispositif fonctionnel additionnel est une fiche électrique femelle.

9. Vaporisateur mural électrique multifonctions selon l'une quelconque des revendications précédentes, dans lequel ledit dispositif fonctionnel additionnel est un témoin de fonctionnement.

10. Vaporisateur mural électrique multifonctions selon l'une quelconque des revendications précédentes, dans lequel ledit dispositif fonctionnel additionnel est un ventilateur.

11. Vaporisateur mural électrique multifonctions selon l'une quelconque des revendications précédentes, dans lequel ledit dispositif fonctionnel additionnel est un éclairage nocturne.

12. Vaporisateur mural électrique multifonctions selon l'une quelconque des revendications précédentes, dans lequel ledit dispositif fonctionnel additionnel est un programmateur de fonctionnement.

13. Vaporisateur mural électrique multifonctions selon l'une quelconque des revendications précédentes, dans lequel ledit dispositif fonctionnel additionnel est un commutateur pour la mise en marche du vaporisateur.
